(19) Europäisches Patentamt
European Patent Office
Office européen des brevets

(11) Publication number: **0 495 225 A1**

# (12) EUROPEAN PATENT APPLICATION

(21) Application number: **91121779.2**

(51) Int. Cl.5: **C07H 19/073**, //A61K31/70

(22) Date of filing: **01.01.92**

(30) Priority: **15.01.91 US 641213**

(43) Date of publication of application: **22.07.92 Bulletin 92/30**

(84) Designated Contracting States: **AT BE CH DE DK ES FR GB GR IT LI LU NL PT SE**

(71) Applicant: **AMERICAN CYANAMID COMPANY**
**1937 West Main Street P.O. Box 60**
**Stamford Connecticut 06904-0060(US)**

(72) Inventor: **d'Antuono III, Joseph**
**68 Junco Court**
**Three Bridges, New Jersey 08887(US)**
Inventor: **Boop, Donald Carl**
**RR1, Box 572**
**Bloomsbury, New Jersey 08804(US)**
Inventor: **Clauss, Steven L.**
**134 South 7th Avenue**
**Manville, New Jersey 08835(US)**
Inventor: **Considine Jr., John Leo**
**488 Winding Brook Way**
**Bridgewater, New Jersey 08807(US)**
Inventor: **Padmanathan, Thurairajah**
**1 Devon Drive West**
**Piscataway, New Jersey 08854(US)**
Inventor: **Rizzo, Carl J.**
**43 Kosciusko Road**
**Whitehouse Station, New Jersey 08889(US)**
Inventor: **Andrade, John Robert**
**322 Church Street**
**Bound Brook, New Jersey 08805(US)**

(74) Representative: **Wächtershäuser, Günter, Dr.**
**Tal 29**
**W-8000 München 2(DE)**

## (54) Process for the preparation of 3'fluoropyrimidine nucleosides.

(57) An improved process for the synthesis of 3'-fluoropyrimidine nucleosides of the general formula:

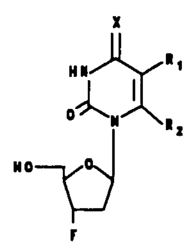

(I)

by reacting a 5'-methanesulfonyl-2'-dideoxy-2,3' -anhydropyrimidine nucleoside at a concentration of up to 20% with hydrogen fluoride in the presence of a suitable aluminum reagent, recovering the 5'-methanesulfonyl-2',3'-dideoxy-3'-fluoropyrimidine nucleoside intermediate by direct crystallization and removing the 5'-methanesulfonyl protecting group in a single step by reaction with an aqueous base solution.

EP 0 495 225 A1

## BACKGROUND OF THE INVENTION

1. Field of the Invention

The present invention is directed to a novel process for the synthesis of 3'-fluoropyrimidine nucleosides, particularly 3'-deoxy-3'-fluorothymidine (FLT) from the corresponding pyrimidine nucleosides such as thymidine.

2. Description of Prior Art

Acquired Immunodeficiency Syndrome (AIDS), recognized as a systemic immunosuppressive disorder, is an infectious disease caused by a retrovirus termed human immunodeficiency virus (HIV). Since HIV is a retrovirvs, viral reverse transcriptase appears to be a selective target for antiviral agents. Accordingly, a number of different reverse transcriptase inhibitors having different chemical structures have been reported to be active against HIV replication in vitro and in vivo.

Of these reverse transcriptase inhibitors, the 2',3'-dideoxyribonucleosides in particular are reported to have significant inhibitory activity against HIV in vitro (R. Dagani, Chem. and Eng. News, 41-49, Nov. 23, 1987; E. De Clercq, A. Van Aerschot, P. Herdewijn, M. Baba, R. Pauwels and J. Balzarini Nucleosides and Nucleotides, 8 (5 and 6), 659-671 (1989); A. Van Aerschot, P. Herdewijn, J. Balzarini, R. Pauwels and E. De Clercq, J. Med. Chem. 32, 1743-1749 (1989)).

Among the 2',3'-dideoxyribonucleoside products reported, 3'-azido-2',3'-dideoxythymidine (AZT), and 3'-deoxy-3'-fluorothymidine (also referred to as 2',3'-dideoxy-3'-fluorothymidine or FLT) in particular show selective anti-HIV activity. The compound 3'-azido-2',3'-dideoxythymidine (AZT) is being sold commercially as a potent inhibitor of HIV-induced cytopathogenicity. However, 3'-deoxy-3'-fluorothymidine is reported to have increased activity over AZT (Balzarini, J., et al., Biochem. Pharmacol. 1988, 37, 2847; P. Herdewijn, J., et al., J. Med. Chem. 30, 1270-1278 (1987)). Accordingly, the compound 3'-deoxy-3'-fluorothymidine (FLT) and other 2' or 3'-fluoro-substituted deoxynucleosides are of particular interest as possible agents for the treatment for AIDS.

3'-Deoxy-3'-fluorothymidine (FLT) has been prepared by Langen et al., by several different routes. The first of these synthetic routes involves the formation of a 2,3'-anhydro derivative of thymidine and its reaction with HF, in the presence of aluminum trifluoride, or with $KHF_2$ or $NH_4F$; Tetrahedron 27(1971) pp. 2463-2472, U.S. Patent No. 3,775,397. The second of these routes involves the formation of 3'-methanesulfonylthymidine which is then reacted with $KHF_2$ or $NH_4F$ to obtain the desired product, see U.S. Patent No. 3,775,397. The third method involves the formation of the 5'-protected-2,3'-anhydro nucleoside derivative of thymidine and its reaction with HF in the presence of aluminum trifluoride followed by a two or three step removal of the 5'-protecting group; see Nucleic Acid Chemistry, Part 1, John Wiley & Sons (1978) pp. 299-302, Townsend and Tipson eds.; J. Prakt. Chem. 315, pp. 895-900 (1973); GDR patent, DD103241, January 12, 1974, Etzold et al.

Other closely related compounds have been fluorinated using diethylaminosulfur trifluoride (DAST) (See A. Van Aerschat et al., J. Med. Chem. 32, pp. 1743-1749 (1989).

The recent discovery of the activity of FLT as an anti-HIV agent has prompted a need for a process which allows the compound to be prepared economically and efficiently on a large scale. However, all of the prior art routes to synthesize FLT are laboratory scale and are not amenable to large scale manufacture of the compound.

The Langen procedure to produce FLT directly from the 2,3'-anhydroderivative of thymidine or the 3'-methanesulfonylthymidine derivative gives very poor yields with extensive cleavage to thymine. The alternative Langen procedure to produce FLT from the 5'-protected-2,3'-anhydro nucleoside derivative of thymidine is not feasible on a large scale in the manner described by Langen. The productivity of both the formation of the anhydro derivative and the fluorination reactions is very low, that is, low concentrations of substrate are required in relation to the amount of solvent and reagents. In addition, the fluorination step requires chromatography and evaporation to dryness to isolate the pure product, which are not practical or desirable on a large scale. Moreover, the removal of the 5'-protecting group involves a complex two or three step procedure in which an acetyl derivative is formed and requires organic solvents, chromatography and evaporation to dryness, all of which are not practical or desirable on a large scale.

## SUMMARY OF THE INVENTION

This invention is an improved process for producing 2',3'-dideoxy-3'-fluoropyrimidine nucleosides of the

formula:

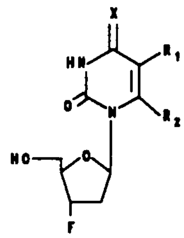

(I)

wherein X is oxygen or sulfur and $R_1$ and $R_2$ may be the same or different and are selected from hydrogen, lower alkyl, halogen, substituted lower alkyl, OH or SH, which process is feasible in operation on a multigram scale and which produces the product in high yields in an easily recoverable manner. The improved process provides for direct crystallization of the intermediates from reaction media and overall simplicity in operation which makes the process suitable for large scale manufacture. The term "lower alkyl" as used herein refers to alkyl groups of 1-4 carbon atoms. The term "substituted lower alkyl" refers to alkyl groups of 1-4 carbon atoms wherein the substituents are halogen or hydroxy.

More specifically, in one aspect, the present invention provides an improved procedure for converting 5'-methanesulfonyl-2'-deoxy-2,3'-anhydrothymidine to 5'-methanesulfonyl-2',3'-dideoxy-3'-fluorothymidine by heating a slurry of the anhydro compound with HF in the presence of a suitable aluminum reagent in an appropriate solvent wherein the concentration of anhydro compound is from 2% up to 20%.

Additionally, the present invention provides a novel single step procedure for removing the 5'-methanesulfonyl protecting group from the nucleoside by reacting the protected nucleoside with an aqueous base solution, acidifying, concentrating to reduce the volume and recovering the product by filtration.

## DETAILED DESCRIPTION OF THE INVENTION

The process for preparing 3'-fluoropyrimidine nucleosides of formula I may conveniently be summarized by the following reaction sequence of Scheme I.

Scheme I

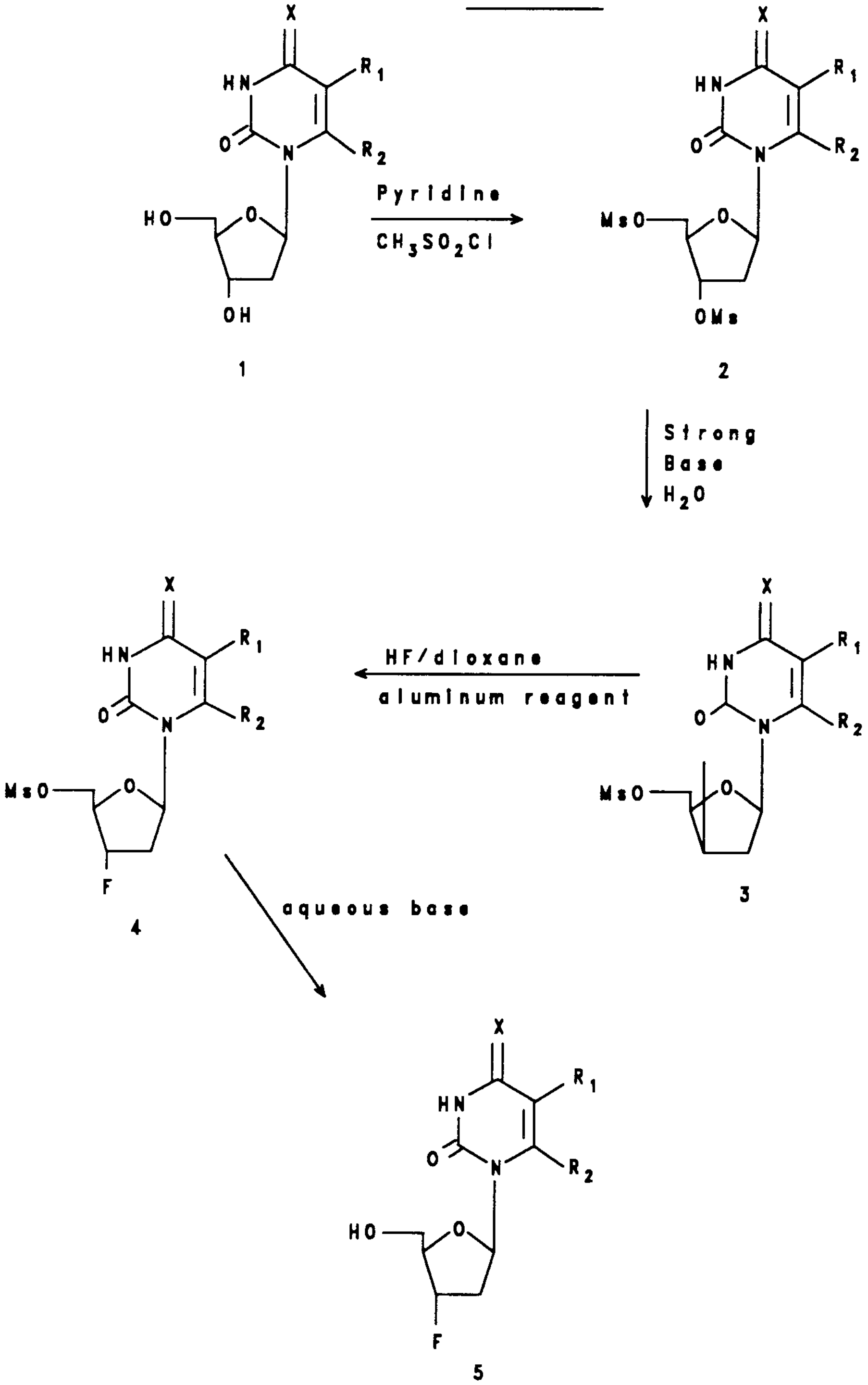


The present invention relates to an improved method of preparing 3'-fluoropyrimidine nucleosides of formula I from the corresponding pyrimidine nucleosides. More particularly, the present invention relates to an improved technique for preparing 3'-deoxy--3'-fluorothymidine (FLT) (5, where X = O, $R_1$ = $CH_3$, $R_2$ = H) from thymidine (1, where X = O, $R_1$ = $CH_3$, $R_2$ = H).

Although the following description refers to thymidine (1, where $R_1$ = $CH_3$, $R_2$ = H and X = O), it is to be understood that any substituted pyrimidine nucleoside compound of formula 1 may be substituted for thymidine in the reaction set forth in Scheme I.

According to the present invention, as set forth in Scheme I, 2',3'-dideoxy-3'-fluoronucleosides of formula 5 are prepared by an improved process comprising the steps of:

(a) converting a thymidine compound of formula 1 to a reactive 3',5'-dimethanesulfonyl intermediate of formula 2 by dissolving the thymidine in pyridine, cooling to 0-5°C, adding methanesulfonyl chloride, allowing the temperature to rise to 20-30°C,cooling, adding water and isolating the 3',5'-dimethanesulfonyl compound by filtration;

(b) reacting an aqueous solution of the 3',5'-dimethanesulfonyl compound in a concentration of about 25% with an acceptable strong base and heating to a temperature of about 50-55°C, cooling and collecting the solid 5'-methanesulfonyl-2,3'-anhydrothymidine compound 3 by filtration;

(c) converting the 5'-methanesulfonyl-2,3'-anhydrothymidine compound 3 to the 3'-fluoro-5'-methanesulfonylthymidine compound 4 by heating a slurry of the compound 3 at a concentration of from 2% up to 20% with HF in the presence of a suitable aluminum reagent at a temperature of about 55-115°C and recovering the 3'-fluoro-5'-methanesulfonylthymidine compound 4 from the reaction mixture without chromatography by quenching the reaction mixture with water and calcium carbonate, filtering, concentrating the mixture to reduce the volume and removing the solid product by filtration; then

(d) reacting the 3'-fluoro-5'-methanesulfonylthymidine compound 4 with aqueous base solution, acidifying, concentrating to reduce the volume and collecting the 3'-deoxy-3'-fluorothymidine compound 5 by filtration.

The invention in the process according to this invention is in the selection, handling and processing of the reactants, intermediates and products in a manner which provides the ability to manufacture 3'-deoxy-3'-fluorothymidine in multi-kilogram quantities using large scale manufacturing equipment. The process is simple, efficient and does not require exotic or exceptionally hazardous materials or conditions.

The improvement in the first step, involving the conversion of thymidine 1 to 3',5'-dimethanesulfonylthymidine 2 is in the reaction conditions employed. The present inventors have found that the reaction time may be reduced from more than 18 hours to about one hour by allowing the reaction temperature to rise to 20-40°C instead of 5°C as employed in the prior art. Preferably, the thymidine compound (1) is dissolved in a basic organic solvent such as pyridine, preferably at 0-5°C, and methanesulfonyl chloride, in a molar ratio of 3 to 1, is added with the temperature rising to 20-40°C. The reaction mixture is stirred at about 20-40°C for about one hour. Cooling, dilution of the mixture with water, filtering the product and drying the solid gives consistent high yields of 2 without the necessity of any additional purification.

The process to prepare the methanesulfonyl anhydro derivative 3 from the 3',5'-di-methanesulfonylthymidine compound 2 has been generally disclosed. However, the present improved process is more suitable for use on a large-scale because it utilizes much higher concentrations of substrate thereby increasing the productivity of the reaction. Additionally, the anhydro formation is carried out in water thereby eliminating the need for organic solvents. Also, the product crystallizes directly from solution. Preferably, 2 is dissolved at a concentration of up to 25% in an aqueous solution of an acceptable base such as 50% aqueous sodium hydroxide and heated at 50-55°C,cooled and compound 3 isolated by filtration. Subsequently, 3 may be stirred with methanol at a concentration of 0.2 g/ml to afford a recovery of at least 97% and a HPLC purity of at least 99%. Surprisingly, the present inventors have found that by carrying out the reaction in water at 50-55°C, a higher concentration (25% as opposed to 1-2%) of 2 may be used without the risk of significant formation of the 3',5'-anhydrothymidine or other by-products.

The present process also provides improvements in preparing 4 from 3. The present improved process is more suitable for use on a large-scale and is more capable of consistently producing a high yield of a relatively pure product. The improvement in this step is in both the fluorination conditions and in the isolation and recovery of the 3'-fluoro-5'-methanesulfonylthymidine product. Prior to the present invention, it was believed by those skilled in the art, that it was necessary to use a very low concentration of substrate, 5'-methanesulfonyl-2,3'-anhydrothymidine (0.5%), when using HF as the fluorination reagent since higher concentrations of substrate would lead to the formation of the 3',5'-dimethanesulfonylthymidine by-product. On the other hand, it was believed that increasing the concentration of HF would cleave the nucleoside from the base and lead to formation of the thymine by-product. The present inventors have found that substrate concentrations of 30-40 times those of the prior art, resulting in a proportionate increase in the productivity of the reaction, may be used provided a critical substrate/HF concentration is employed. When aluminum trifluoride is used as the reagent, the critical ratio is 1:2 substrate to HF on a molar basis.

According to the present invention, a slurry of substrate, 5'-methanesulfonyl-2,3'-anhydrothymidine 3, at a concentration of 2% up to 20%, in an appropriate solvent, is heated at a temperature of 55-115°C in the presence of a suitable aluminum reagent and HF. Preferably, the HF is first dissolved in a suitable inert solvent at concentrations of up to 20%.

The aluminum reagent used may be dried aluminum trifluoride. Commercially available "Anhydrous" aluminum trifluoride is generally unsuitable because it gives inconsistent results. Best results are obtained when aluminum trifluoride hydrate or trihydrate is dried to a weight loss of 25-35% in a forced air oven at

120-180°C prior to being added to the reaction mixture.

Preferably, the aluminum reagent used is a substituted organo-aluminum reagent. For example, aluminum isopropoxide, trihexyl aluminum, diethyl-aluminum fluoride and aluminum acetylacetonate may be used as reagents. Most preferably, aluminum acetylacetonate is used in molar ratios of 1.0 to 3.0 relative to the substrate. In the event the substituted organo aluminum reagent such as aluminum acetylacetonate is employed in the reaction mixture, the concentration of HF is increased such that there are 4-6 moles of fluoride ion to each mole of substrate.

In a preferred embodiment, an inorganic alkalimetal hydrogen fluoride compound of the formula $MHF_2$ where M is $NH_4$, K, Na or Li is added to the reaction mixture. For example, a substituted organo-aluminum reagent as discussed above such as aluminum acetylacetonate is employed in molar proportions of 1.0-3.0 relative to the substrate and 0.2 to 4 molar proportions of the alkali metal hydrogen fluoride compound is added to the reaction mixture with 4-6 molar proportions of hydrogen fluoride. In a specific embodiment, the alkali metal hydrogen fluoride compound is ammonium hydrogen difluoride in molar proportions of 0.5 to 1.0 molar equivalents.

As stated above, the fluorination reaction is carried out in an inert solvent. Suitable inert solvents which may be used include tetrahydrofuran, acetone, dioxane, chloroform, dichloromethane, ether, nitrobenzene, dimethylsulfoxide, 1,2-dichloroethane, 1,2-dimethoxyethane, toluene and acetonitrile and/or any combination thereof. Preferably, the solvent is dioxane.

The improvement in the process of preparing the 3'-fluoro-5'-methanesulfonylthymidine compound 4 also resides in the isolation and recovery of the fluorinated protected nucleoside. According to the improved process, the pure product is isolated as a filterable solid because it crystallizes directly out of solution and no chromatography is required to obtain a high level of purity. In addition, the procedure does not require evaporation to dryness, an operation which is not practical or desirable on an industrial scale.

Accordingly, following reaction of the 5'-methanesulfonyl-2,3'-anhydrothymidine compound 3 with the HF/aluminum compound fluorination mixture at 55-115°C, the reaction mixture is cooled to ambient temperature and drowned in a slurry of water and calcium carbonate. After stirring, the slurry is filtered, washed with acetone or water, and the combined filtrate and wash concentrated to reduce the volume. The pure 3'-fluoro-5'-methanesulfonylthymidine 4 product is then recovered from the mixture by filtration. A highly pure product (≧95% by HPLC) is thereby obtained without the need for chromatography because the product crystallizes directly out of solution.

In the final step of the reaction, in which the 5'-methanesulfonyl protecting group is removed from the 3'-fluoro-5'-methanesulfonylthymidine compound 4 to yield the final product, 3'-deoxy-3'-fluorothymidine 5, the improvement of the present invention provides a simple single step procedure for the removal of the protecting group. The prior art only disclosed a complicated two step process in which 4 is first acylated with acetic anhydride and then cleaved with alcohol/ammonia. The product is purified by chromatography. In contrast, the present improved process is more suitable for use on a large-scale and is more capable of consistently producing a high yield of relatively pure product. The conversion is accomplished in one step, without the need of organic solvents or chromatography for purification. The direct conversion of 4 to 5 is unique and, surprisingly, does not lead to any appreciable amounts of elimination or other by-products. Additionally, the single step procedure results in improved yields of greater than 70% isolated product as compared to about 40% yield of isolated product using the multi-step procedures of the prior art. Removal of the methanesulfonyl blocking group is best accomplished simply and directly without the use of organic solvents by stirring 4 with aqueous base solutions such as an alkali metal hydroxide or carbonate, acidifying, concentrating to reduce the volume, and collecting the product by filtration. For example, 4 may be reacted with sodium or potassium hydroxide for 1.5-5 hours at 55-95°C, preferably 60-70°C. Adjustment of the pH to 4-5 with aqueous acetic acid, partial concentration, followed by cooling and filtering, gives 3'-deoxy-3'-fluorothymidine (FLT) (5) in up to 77% yield and a purity of at least 98% as shown by HPLC. The demesylation proceeds in greater than 90% conversion as evidenced by HPLC analysis. Suitable aqueous base solutions include aqueous alkali hydroxides such as sodium hydroxide, potassium hydroxide, ammonium hydroxide, lithium hydroxide, tetra alkyl ammonium hydroxide and the like. The corresponding carbonates are also suitable.

Upon further study of the specification and appended claims, further objects and advantages of this invention will become apparent to those skilled in the art.

This invention will be described in greater detail in conjunction with the following, non-limiting, specific examples.

Example 1

3',5'-Dimethanesulfonylthymidine

A stirred mixture of 4.13 kg of thymidine and 19.2 L of pyridine is cooled to 0-5°C and 5.9 kg of methanesulfonyl chloride added at a rate which allows the temperature to rise to 20-30°C. Following an additional hour of stirring, the mixture is cooled to 0-5°C and 29.4 L of water is added while allowing the temperature to rise to 20-30°C. The mixture is cooled to 0-5°C and stirred for 15-30 minutes. The resulting solid is recovered by filtration, and the cake washed with 17 L of water. The solid is dried in a forced air oven at ambient temperature to give 6.526 kg of the desired product as a solid, m.p. 162.2-164.9°C. $^1$H NMR ($d_6$-DMSO) $\delta$ 11.40(s,1H), 7.52(s,1H), 6.23(t,J = 7.07 Hz,1H), 5.31(m,1H), 4.46(m,2H), 4.38(m,1H), 3.33-(s,3H), 3.26(s,3H), 2.52(m,2H), 1.79(s,3H); FTIR (NUJOL) 3156, 3091, 1712, 1674, 1347, 1170, 956, 936, 838 $cm^{-1}$. The purity is 98.6% as shown by HPLC.

Example 2

5,-Methanesulfonyl-2,3'-anhydrothymidine

A stirred mixture of 9.165 kg of 3',5'-dimethanesulfonylthymidine, 45.7 L of water and 18.36 kg of sodium hydroxide, 50%, is heated to 50-55°C. An additional 184 g of sodium hydroxide, 50%, is added and heating continued for an additional hour at 50-55°C. The mixture is cooled to 0-5°C and stirring continued for 15-30 minutes. The resulting solid is collected by filtration, the cake washed with 4.6 L of 3A alcohol and dried to give 6.222 kg of the desired product, m.p. 167.9-169.4. Purity by HPLC is 98.9%. $^1$H NMR ($d_6$-DMSO) $\delta$ 7.60(s,1H), 5.91(m,1H), 5.36(m,1H), 4.49(m,2H), 4.22(m,1H), 3.20(s,3H), 2.60(m,2H), 1.76(s,3H), FTIR (NUJOL) 1664, 1610, 1525, 1465, 1356, 1180, 1163, 980, 851 $cm^{-1}$. A slurry of 5.68 kg of product in 28.4 L of methyl alcohol is stirred for 1 hour. The solid is isolated by filtration and the cake washed with 5.6 L of methyl alcohol. The cake is dried in a forced air oven at 40-50°C to give 5.488 kg of product with 99% purity by HPLC.

Example 3

3,-Fluoro-5'-methanesulfonylthymidine

To a stirred clave is charged 8 g of 5'-methanesulfonyl-2'-deoxy-2,3'-anhydrothymidine, 90 mL of dioxane, 32 g of aluminum trifluoride trihydrate (dried in a forced-air oven at 120-180°C to a loss on drying of 30%) and 10 mL of 10% HF in dioxane. The clave is sealed and heated at 88-90°C for 3 hours. Upon cooling to ambient temperature, the batch is drowned in a slurry of 30 g of calcium carbonate in 75 mL of water and stirred for 25 minutes. The slurry is filtered and the cake washed with acetone (5 x 25 mL). The combined filtrates are evaporated to give 8.22 g of the desired product, with an HPLC purity of 73%, m.p. 144.9-151.9°C. $^1$H NMR ($CDCl_3$) $\delta$ 11.40(s,1H), 7.52(s,1H), 6.24(dd,J = 8.9 Hz,6.0 Hz,1H), 5.37(dd,J = 53.3 Hz,4.2 Hz,1H), 4.44(s,2H), 4.42(m,1H), 3.26(s,3H), 2.40(m,2H), 1.78(s,3H).

Example 4

3'-Fluoro-5'-methanesulfonylthymidine

To a stirred clave is charged 15 L of dioxane, 600 g of 5'-methanesulfonyl-2'-deoxy-2,3'-anhydrothymidine, 1200 g of aluminum trifluoride trihydrate (dried in a forced-air oven at 120-180°C to a loss on drying of 30%) and 760 mL of 10% HF in dioxane. The clave is sealed and heated at 85-95°C for 3 hours. After cooling to ambient temperature, the batch is drowned in a stirred slurry of 470 g of calcium carbonate and 6 L of water. The slurry is stirred for 15-30 minutes and filtered through a bed of diatomaceous earth. The cake is washed with 6 L of acetone and the combined filtrate and washes concentrated under vacuum to 5-10 L followed by the addition of 2 L of water and further concentration to approximately 2.5 L. The mixture is cooled at 5-10°C and the resulting solid filtered. The cake is washed with 1 L of cold water and dried to constant weight to give 450 g of the desired product. HPLC purity of 96.5%.

Example 5

3'-Fluoro-5'-methanesulfonylthymidine

To a stirred autoclave is charged 25 g of 5'-methanesulfonyl-2'-deoxy-2,3'-anhydrothymidine, 83 mL of 2M trihexyl aluminum in dioxane, 98 mL of dioxane and 19 mL of a mixture of 70% hydrogen fluoride and 30% pyridine. The clave is sealed, heated to 85-90°C and stirred at approximately 90°C for 3 hours. The batch is cooled to room temperature and drowned in a mixture of calcium carbonate (40 g) in water (100 mL). The mixture is stirred for about 15 minutes, clarified and the cake washed with acetone (4 X 25 mL). The solution is partially concentrated under vacuum, water is added (200 mL) and the solution concentrated further. The mixture is cooled to 0-5°C, filtered, washed with cold water (about 45 mL) and dried to yield 22.2 g of product.

Example 6

3'-Fluoro-5'-methanesulfonylthymidine

To a stirred autoclave is charged 1,000 g 5'-methanesulfonyl-2'-deoxy-2,3'-anhydrothymidine, 6,000 mL of 1,4-dioxane, 1,180 g of aluminum acetylacetonate, 100 g of ammonium hydrogen difluoride and 4,000 mL of a 10% solution of hydrogen fluoride in dioxane. The clave is sealed and heated at 85-90°C for 3 hours. The batch is cooled to 20-30° and drowned into a slurry of calcium carbonate (2,000 g) in water (10,000 mL). The slurry is stirred for 15-30 minutes and the solids removed by filtration. The filter cake is washed with acetone (7,500 mL) and the combined filtrate and wash is concentrated under reduced pressure to a volume of 6-7.5 liters. Then water (2,000 mL) is added and the solution is concentrated further to 7-7.5 L. The mixture is cooled to 0-5°C and stirred at 0-5°C for 30-60 minutes. The product is filtered, washed with cold water (1,500 mL) and dried to yield 762 g of 5'-methanesulfonyl-2',3'-dideoxy-3'-fluorothymidine.

Example 7

3'-Deoxy-3'-fluorothymidine

To a reaction flask with stirring is charged 31.84 L of water, 1,496 g of 85% potassium hydroxide pellets and 3,184 g of 5'-methanesulfonyl-2',3'-dideoxy-3'-fluorothymidine.The solution is heated at 63-67°C for 3 hours. The pH is adjusted to 4.2-4.7 with 50% aqueous acetic acid and 318 g of activated carbon and 318 g of diatomaceous earth added followed by an additional hour of stirring at 63-67°C. The mixture is filtered through a pad of 318 g of diatomaceous earth and the cake washed with 6.4 L of hot water. The combined filtrates are evaporated in vacuo to 9.6 L and the resulting slurry cooled to 0-5°C. The solids are isolated by filtration and the cake washed with 6.4 L of cold water. The wet cake is dried in an oven to give 1.526 kg of the desired product, m.p. 173.3-177.7°C. HPLC purity 99.5%. $^{1}H$ NMR($d_6$-DMSO) $\delta$ 11.35(s,1H), 7.7(s,1H), 6.22(dd, J = 9.1 Hz, 5.6 Hz,1H), 5.32(dd, J = 53.9 Hz,4.1 Hz,1H), 5.21(s,1H), 4.15(dt, J = 28.0 Hz, 3.8 Hz,1H), 3.68-3.55(m,2H), 2.5-2.2(m,2H); Mass Spec. $M^+$ = 244; FTIR(Nujol) 3425, 3100, 1283, 1109, 995, 870, 850 $cm^{-1}$.

Example 8

3'-Deoxy-3'-fluorothymidine

To a reaction flask, with stirring, is charged 100 mL of water, 2.85 g of sodium hydroxide pellets and 10.0 g of 5'-methanesulfonyl-2',3'-dideoxy-3'-fluorothymidine. The reaction mixture is heated and stirred at 63-67°C for 3 hours. The pH is adjusted to 4.4 by adding 12.8 g of 50% aqueous acetic acid followed by the addition of 1 g of activated carbon and 1 g of diatomaceous earth with stirring continued at 63-67°C for 1 hour. The mixture is filtered and the cake washed with 20 mL of hot water. The combined filtrates are concentrated to 30 mL in vacuo and the resulting slurry cooled to 0-5°C. The solids are isolated by filtration and the cake washed with 20 mL of cold water. The wet cake is dried in a vacuum oven at 45-50°C to give 5.87 g of 3'-deoxy-3'-fluorothymidine. HPLC purity of 98.3%.

Example 9

3'-Deoxy-3'-fluorothymidine

To a reaction flask with stirring is charged 100 mL of water, 4.70 g of 85% potassium hydroxide pellets and 10.0 g of 5'-methanesulfonyl-2',3'-dideoxy-3'-fluorothymidine. The solution is heated at 63-67°C for 3

hours. The pH is adjusted to 4.2-4.7 with 12.8 g of 50% aqueous acetic acid followed by the addition of 1 g of activated carbon and 1 g of diatomaceous earth with stirring continued at 63-67°C for one hour. The mixture is filtered through a pad of 1 g of diatomaceous earth and the cake washed with 20 mL of hot water. The combined filtrates are evaporated in vacuo to 30 mL and the resulting slurry cooled to 0-5°C. The solids are isolated by filtration and the cake washed with 20 mL of cold water. The wet cake is dried in an oven to give 5.62 g of the desired product, m.p. 173.3-177.7°C. HPLC purity 98.0%.

**Claims**

**1.** A process for producing 2',3'-dideoxy-3'-fluoropyrimidine nucleosides of the formula:

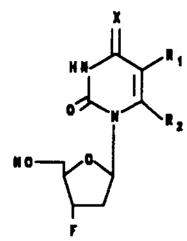

(I)

wherein X is oxygen or sulfur, $R_1$ and $R_2$ may be the same or different and are selected from hydrogen, lower alkyl ($C_1$-$C_4$), substituted lower alkyl ($C_1$-$C_4$) (wherein the substituents are halogen or hydroxy), halogen, OH or SH which comprises the steps of:

(a) dissolving a pyrimidine nucleoside of the formula:

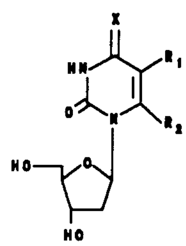

in pyridine, cooling to 0-5°C, adding methanesulfonyl chloride in a molar ratio of 3 to 1 methanesulfonyl chloride to nucleoside, allowing the temperature to rise to 20-40°C, cooling, adding water and isolating a 3',5'-di-methanesulfonylpyrimidine nucleoside compound by filtration; then

(b) reacting an aqueous solution of the 3',5'-dimethanesulfonylpyrimidine nucleoside compound in a concentration of up to 25% with an acceptable strong base and heating to a temperature of about 50-55°C, cooling and collecting a solid 5'-methanesulfonyl-2'-dideoxy-2,3'-anhydropyrimidine nucleoside compound by filtration; then

(c) converting the 5'-methanesulfonyl-2'-dideoxy-2,-3'-anhydropyrimidine nucleoside compound to a 5'-methanesulfonyl-2',3'-dideoxy-3'-fluoropyrimidine nucleoside compound by heating a slurry of the anhydropyrimidine nucleoside compound at a concentration of from 2% up to 20% with hydrogen fluoride in the presence of a suitable aluminum reagent in sufficient concentration such that there are 2 to 6 moles of fluoride to each mole of anhydro pyrimidine nucleoside at a temperature of about 55-115°C and recovering the 5'-methanesulfonyl-2',3'-dideoxy-3'-fluoro-pyrimidine nucleoside compound by treating the reaction mixture with water and calcium carbonate, filtering, concentrating the mixture to a reduced volume and isolating the solid product by filtration; then

(d) reacting the 5'-methanesulfonyl-2',3'-dideoxy-3'-fluoropyrimidine nucleoside compound with aqueous base, acidifying to a pH of 4 to 5, concentrating to a reduced volume, and collecting the 3'-deoxy-3'-fluoropyrimidine nucleoside compound by filtration.

**2.** The process of claim 1 wherein the 2',3'-dideoxy-3'-fluoropyrimidine nucleoside compound is 3'-deoxy-3'-fluorothymidine.

**3.** The process of claim 1 wherein the acceptable strong base in step (b) is sodium hydroxide or potassium hydroxide.

**4.** The process of claim 1 wherein the suitable aluminum reagent of step (c) is heat treated aluminum trifluoride hydrate.

**5.** The process of claim 1 wherein the suitable aluminum reagent of step (c) is a substituted organoaluminum compound selected from the group consisting of aluminum acetylacetonate, trihexyl aluminum and aluminum isopropoxide.

**6.** The process of claim 1, step (c), wherein an inorganic alkali metal hydrogen fluoride compound of the formula $MHF_2$ where M is $NH_4$, K, Na or Li is added to the reaction mixture.

**7.** In a process for producing 3'-deoxy-3'-fluorothymidine (FLT) wherein an intermediate 5'-methanesulfonyl-2,3'-anhydrothymidine is prepared from 3',5'-dimethanesulfonylthymidine, the improvement comprising reacting 3',5'-dimethanesulfonylthymidine in concentrations of up to 25% in aqueous sodium hydroxide at 50-55°C for 1 hour, cooling to 0-5°C, stirring and collecting the solid by filtration.

**8.** In a process for producing 3'-deoxy-3'-fluorothymidine wherein an intermediate, 3'-fluoro-5'-methanesulfonylthymidine is formed from 5'-methanesulfonyl-2,3'-anhydrothymidine by reaction with HF in the presence of dried aluminum fluoride hydrate, the improvement which comprises heating a slurry of the 5'-methanesulfonyl-2,3'-anhydrothymidine in the presence of dried aluminum trifluoride in an appropriate solvent wherein the concentration ratio of HF to 5'-methanesulfonyl-2,3'-anhydrothymidine is about 2:1 on a molar basis, at a temperature of 80-115°C for about 3 hours and recovering the intermediate.

**9.** In a process for producing 3'-deoxy-3'-fluorothymidine wherein an intermediate 3'-fluoro-5'-methanesulfonylthymidine is prepared from 5'-methanesulfonyl-2,3'-anhydrothymidine, the improvement comprising, recovering the purified intermediate from the reaction mixture formed by reaction of 5'-methanesulfonyl-2,3'-anhydrothymidine with HF in the presence of a suitable aluminum reagent by stirring the reaction mixture with water and calcium carbonate, filtering, concentrating to a reduced volume and isolating the solid product by filtration.

**10.** In a process for producing 3'-deoxy-3'-fluorothymidine (FLT) from an intermediate, 3'-fluoro-5'-methanesulfonylthymidine, the improvement comprising reacting the 3'-fluoro-5'-methanesulfonylthymidine with agueous base, acidifying, concentrating to a reduced volume and collecting the product by filtration.

European Patent Office

# EUROPEAN SEARCH REPORT

Application Number: EP 91121779.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | CHEMICAL ABSTRACTS, vol. 115, no. 7, August 19, 1991, Columbus, Ohio, USA K. GREEN et al. " Hydro-fluorination of anhydro-thymidine via soluble aluminium derivates" page 853, abstract-no. 72 128p & Tetrahedron Lett. 1991, 32(9), 2091-4 -- | 1,2,8 | C 07 H 19/073 //A 61 K 31/70 |
| D,A | DD - A - 103 241 (G. ETZOLD et al.) * Examples 1-3 * -- | 1,8 | |
| D,A | US - A - 3 775 397 (G. ETZOLD et al.) * Examples 1-7 * ---- | 1,8 | |

TECHNICAL FIELDS SEARCHED (Int. Cl.5)

C 07 H
A 61 K

The present search report has been drawn up for all claims

| Place of search | Date of completion of the search | Examiner |
|---|---|---|
| VIENNA | 14-04-1992 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document